# EUROPÄISCHE PATENTSCHRIFT

(11) **EP 0 705 905 B1**
(45) Veröffentlichungstag und Bekanntmachung des Hinweises auf die Patenterteilung: **10.10.2001**
(21) Anmeldenummer: 95110768.9
(22) Anmeldetag: 11.07.1995
(51) Int. Cl.: C12Q 1/68

(54) **Verfahren zum sensitiven Nachweis von Nukleinsäuren**
Method for the sensitive detection of nucleic acids
Procédé pour la détection sensible d'acides nucléiques

(30) Priorität: 16.07.1994 DE 4425264; 02.09.1994 DE 4431269
(43) Veröffentlichungstag der Anmeldung: 10.04.1996
(73) Patentinhaber: Roche Diagnostics GmbH, 68298 Mannheim (DE)
(72) Erfinder: Rosemeyer, Viola, B-1300 Wavre (BE); Seibl, Rudolf, Dr., D-82377 Penzberg (DE)

(56) Entgegenhaltungen:
- EP-A- 0 300 796
- EP-A- 0 718 408
- WO-A-89/09284
- WO-A-93/06240

## Beschreibung

Gegenstand der Erfindung ist ein Verfahren zum sensitiven Nachweis von Nukleinsäuren durch Hybridisierung einer Sondennukleinsäure mit einer Zielnukleinsäure, Abbau des hybridisierten Teils der Sondennukleinsäure und Nachweis des Spaltprodukts, sowie ein hierfür geeignetes Set von Reagenzien.

Die Nukleinsäurehybridisierung hat aufgrund Ihrer Spezifität in molekularen Analysen weite Verbreitung gefunden. Eine Vielzahl unterschiedlicher Amplifikationsmethoden wurden mit der Zielsetzung entwickelt, die oft nicht ausreichende Sensitivität konventioneller Detektionsverfahren zum Nachweis der Sondenmoleküle zu steigern. Dabei wurden sowohl Verfahren eingesetzt die auf einer Vermehrung der Zielsequenz beruhten, aber auch solche, die auf einer Zielsequenz-spezifischen Signalverstärkung beruhen. Ein Beispiel der Vermehrung der Zielsequenz ist die in EP-A-0 200 362 beschriebene Polymerasekettenreaktion, in der die beiden Stränge der Zielnukleinsäure in einer in-vitro Replikationsreaktion amplifiziert werden. Die Temperatur der Reaktion wird zyklisch verändert, um eine doppelsträngige Zielnukleinsäure zu denaturieren, die Hybridisierung mit Initiatormolekülen und die anschließende DNA-Synthese zu ermöglichen und diese Schritte zu wiederholen.

Für die Vermehrung von Zielsequenzen wurden jedoch auch in-vitro Transkriptionen verwendet. Ein Beispiel für eine solche Reaktion ist in WO 88/10315 beschrieben. Bei dieser Amplifikation werden ebenfalls zwei Primer eingesetzt, von denen jedoch einer eine Promotersequenz enthält. Die Primer sind zu unterschiedlichen Strängen der zu amplifizierenden Sequenz komplementär. In EP-A-0 329 822 ist eine Weiterentwicklung dieses Verfahrens beschrieben, bei der die durch Transkription des als Zwischenprodukt entstandenen doppelsträngigen Nukleinsäureprodukts entstandenen Ribonukleinsäuren im Hybrid abgebaut werden, um die erneute Hybridisierung der neu synthetisierten DNA mit einem promoterhaltigen Primermolekül zu ermöglichen. Durch den Abbau der ursprünglich gebildeten Transkripte wird es möglich, aus jedem gebildeten RNA Molekül erneut eine doppelsträngige promoterhaltige Nukleinsäure zu bilden, die für einen erneuten Transkriptionsstart verwendet werden kann.

Im Gegensatz zu den vorangehend geschilderten Methoden dient die Zielsequenz-spezifische Signalverstärkung nicht zur Vermehrung der nachzuweisenden Nukleinsäure, sondern hat nur ihre spezifische Identifizierung zum Ziel. Um dies zu erreichen, wird ein Signal in Abhängigkeit von der Anwesenheit der Zielsequenz verstärkt. Eine solche Methode ist in WO 89/09284 und WO 89/10415 beschrieben. In dieser sogenannten Cycling-Probe-Reaktion (CPR) wird ein markiertes, chimäres DNA-RNA-DNA-Sondenmolekül eingesetzt, das mit einem Ziel-DNA-Molekül hybridisiert. Durch die Hybridisierung entsteht ein RNA-DNA-Hybrid, das ein Substrat für die RNAse H darstellt. Da dieses Enzym spezifisch den RNA Anteil des RNA-DNA-Hybrides abbaut, wird das Sondenmolekül gespalten und die entstehenden Fragmente disoziieren aufgrund der geringeren Schmelztemperatur von der Zielsequenz ab. Daraufhin kann das Zielmolekül mit einem weiteren Sondenmolekül hybridisieren und der Zyklus wird wiederholt. Die Fragmente des Sondenmoleküls werden über die daran befindliche Markierung nachgewiesen.

Gegenstand der vorliegenden Erfindung war es, die Empfindlichkeit von Verfahren zu erhöhen, bei denen eine Zielnukleinsäure mit einem Sondenmolekül hybridisiert wird und der Abbau des Sondenmoleküls zur Bestimmung der Zielnukleinsäure verwendet wird.

Gegenstand der Erfindung ist daher ein Verfahren zum empfindlichen Nachweis einer Ziel-nukleinsäure A durch
- Hybridisierung der Zielnukleinsäure A mit einer Sondennukleinsäure B, die mindestens einen mit der Zielnukleinsäure hybridisierenden Teil B1 und einen nicht mit der Zielnukleinsäure hybridisierenden nukleinsäurespezifischen Teil B2 enthält,
- Spaltung der Sondennukleinsäure B im Teil B1,
- Hybridisierung eines Spaltproduktes B' der Sondennukleinsäure B, welches den nicht mit der Zielnukleinsäure hybridisierenden Teil B2 enthält, mit einer Matrizennukleinsäure C, die einen mit dem Spaltprodukt im Teil B2 hybridisierbaren Teil C2 und einen nicht mit dem Teil B1 der Sondennukleinsäure hybridisierbaren Teil C1 enthält, und
- Bestimmung des Hybrids aus Spaltprodukt B' und Matrizennukleinsäure C, sowie ein Reagenzkit zur Durchführung dieses Verfahrens.

Das erfindungsgemäße Verfahren dient zum Nachweis einer Zielnukleinsäure A. Diese Zielnukleinsäure kann beliebigen Ursprungs sein, beispielsweise Nukleinsäuren viralen, bakteriellen oder zellulären Ursprungs. Sie können in Lösung, Suspension aber auch an Festkörpern fixiert oder in zellhaltigen Medien, Zellabstrichen, fixierten Zellen, Gewebsschnitten oder fixierten Organismen vorliegen. Bevorzugt liegen die Nukleinsäuren in Lösung vor.

Üblicherweise beginnen Nachweisverfahren mit einem Schritt zur Verfügbarmachung der Analytnukleinsäure mit entsprechenden Reagenzien. Hierbei können sowohl Veränderungen des pHs (alkalisch), Hitze, Wiederholung extremer Temperaturveränderungen (Einfrieren/Auftauen), Veränderung der physiologischen Wachstumsbedingungen (osmotischer Druck), Einwirkung von Detergentien, chaotropen Salzen oder Enzymen (z.B. Proteasen, Lipasen) allein oder in Kombination zur Freisetzung der Nukleinsäuren beitragen. Da das erfindungsgemäße Verfahren sehr empfindlich und selektiv ist, können auch kleine Nukleinsäuremengen in Anwesenheit anderer Stoffe, beispielsweise Proteinen, Zellen, Zellfragmenten, aber auch nicht nachzuweisender Nukleinsäuren nachgewiesen werden.

Geeignete Zielnukleinsäuren sind beispielsweise Ribonukleinsäuren und Deoxyribonukleinsäuren. Die Nukleinsäuren können auch modifiziert sein, beispielsweise durch vorher vorgenommene Bearbeitungsschritte. Solche Bearbeitungsschritte können beispielsweise einen Verdau von Nukleinsäuren, z.B. durch ein Restriktionsenzym beinhalten. Besonders bevorzugt als Zielnukleinsäure ist Deoxyribonukleinsäure (DNA).

Bei dem erfindungsgemäßen Verfahren handelt es sich um eine spezielle Ausführungsform von auf einem Hybridisierungsereignis beruhenden Test, im Speziellen der zielsequenz-spezifischen Signalverstärkung. Auf Hybridisierungsereignissen beruhende Tests sind die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, z.B. in den Kapiteln 1 (Hybridisation Strategy), 3 (Quantitative Analysis of Solution Hbyridisation) und 4 (Quantitative Filter Hybridisation), Current Protocols in Molecular Biology, Edt. F.M. Ausubel et al., J. Wiley and Son, 1987, und Molecular Cloning, Edt. J. Sambrook et al., CSH, 1989, Bezug genommen. Zu den bekannten Methoden gehört auch die Herstellung von markierten Nukleosidtriphosphaten, wie sie in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Komplentarität zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe L. Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin-, Biotin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Nukleosidtriphosphate (NTP) sind Ribo (rNTP)- oder Desoxyribonukleosidtriphosphate (dNTP).

Als Sondennukleinsäuren B sind Moleküle geeignet, die zwei miteinander verbundene Teile B1 und B2 enthalten. Teil B1 ist dadurch charakterisiert, daß er mit der Zielnukleinsäure bzw. einem Teil davon hybridisieren kann. Dazu ist dieser Teil ausreichend komplementär. Darüberhinaus muß der Teil B1 gespalten werden können, wenn er in mit der Zielnukleinsäure hybridisierter Form vorliegt. Unter Spaltung ist hierbei die Aufteilung des Teiles B1 in zwei oder mehr nicht mehr aneinander gebundene Teile zu verstehen, wobei die Zielnukleinsäure nicht gespalten wird. Teil B1 kann daher z.B. ribonukleotidische oder abasische Sequenzen enthalten. In einem bevorzugten Fall enthält Teil B1 zwei oder mehr über die in Nukleinsäuren übliche Weise miteinander verknüpfte Monoribonukleotideinheiten, während der hierzu komplementäre Teil der Zielnukleinsäure eine Deoxyribonukleinsäure darstellt. In diesem Fall kann eine Spaltung der Sondennukleinsäure B im Teil B1 dadurch geschehen, daß das gebildete Hybrid mit RNAse H in Kontakt gebracht wird. Im Falle der Anwesenheit abasischer Sequenzen kann der Abbau mittels AP-Endonuklease geschehen.

Dabei wird mindestens ein Teil des hybridisierbaren Teiles B1 verdaut. Dadurch bildet sich ein Spaltprodukt B', welches den nicht mit der Zielnukleinsäure hybridisierenden nukleinsäureselektiven Teil B2 sowie eventuelle Teile des ursprünglich mit der Zielnukleinsäure hybridisierten Teiles B1 enthält.

Die Bedingungen für die Hybridisierungen der Zielnukleinsäure mit der Sondennukleinsäure werden vorzugsweise so gewählt, daß gerade noch eine selektive Hybridisierung der Sondennukleinsäure mit der Zielnukleinsäure stattfindet, eine unselektive Hybridisierung der Sondennukleinsäure mit anderen, nicht nachzuweisenden Nukleinsäuren der Probe gerade nicht mehr stattfindet. Die durch die Spaltung der Sondennukleinsäure entstehenden Bruchstücke, darunter auch das Spaltprodukt B', sind kürzer als die ursprüngliche Sondennukleinsäure und werden unter den gewählten Bedingungen daher kein stabiles Hybrid mehr mit der Zielnukleinsäure bilden können. Unter den gewählten Bedingungen werden sie daher die Zielnukleinsäure A freigeben.

Durch Spaltung der Sondennukleinsäure können auch unterschiedliche Bruchstücke B' entstehen, welche entweder nur den Teil B2 alleine enthalten oder aber zusätzlich noch Reste des Teils B1. Dies hängt von den jeweils verwendeten Bedingungen ab.

Unter einem nukleinsäurespezifischen Teil einer Nukleinsäure wird im Folgenden eine Sequenz verstanden, die mit einer anderen Sequenz als der Zielnukleinsäure hybridisieren kann, wobei unter den gewählten Bedingungen eine spezifische Hybridisierung stattfindet, d.h. keine für das Gesamtverfahren relevante Hybridisierung mit weiteren unbeteiligten in der Reaktionsmischung vorhandenen Nukleinsäuren stattfindet. Typische nukleinsäurespezifische Teile sind Teil B2 der Sondennukleinsäure und Teil C2 der Matrizennukleinsäure.

Der nicht mit der Zielnukleinsäure hybridisierende, nukleinsäurespezifische Teil B2 der Sondennukleinsäure muß die Bedingung erfüllen, daß er unter den Bedingungen des zielsequenzspezifischen Abbaus des Teiles B1 nicht abgebaut wird. Außerdem soll er mit dem später definierten Matrizenoligonukleotid hybridisieren können. Die Sequenz des Teils B2 kann prinzipiell frei gewählt werden. Hierbei ist jedoch zu berücksichtigen, daß die Hybridisierung des Spaltprodukts B' mit dem Matrizenoligonukleotid durch eine Komplementarität mit der Zielnukleinsäure erschwert wird, wodurch voraussichtlich die Sensitivität gegenüber dem optimalen Fall erniedrigt ist. Darüber hinaus sollte darauf geachtet werden, daß das Sondenmolekül nicht zur Bildung partieller Doppelstränge neigt. B2 kann ebenfalls eine Ribonukleinsäure, Deoxyribonukleinsäure oder modifizierte Nukleinsäure sein. Für den Fall, daß der hybridisierbare Teil B1 eine Ribonukleinsäure enthält und die Spaltung mittels einer RNAse vorgenommen wird, stellt Teil B2 bevorzugt keine unter diesen Bedingungen spaltbare Ribonukleinsäure dar, bevorzugt stellt er Deoxyribonukleinsäure dar. B2 kann jedoch auch eine so modifizierte Ribonukleinsäure sein, daß sie nicht mehr von einer RNAse gespalten werden kann. Eine weitere Möglichkeit ist die Verwendung von Nukleinsäureanalogen, die zwar noch die Hybridisierungseigenschaften einer Nukleinsäure aufweisen, jedoch keinen Phosphat-Zucker-Kettenanteil haben. Hierfür sind insbesondere PNA-Moleküle der WO 92/20702 oder WO 86/05518 geeignet. Die Bedingung, daß dieser Anteil nicht mit der Zielnukleinsäure hybridisieren soll, bezieht sich insbesondere auf die während der Hybridisierung der Sondennukleinsäure mit der Zielnukleinsäure angelegten Bedingungen. Bevorzugt wird der Teil B2 so gewählt, daß er auch nicht mit nicht nachzuweisenden Nukleinsäuren der Probe hybridisiert. Teil B2 soll jedoch eine Sequenz beinhalten, die mit der Matrizennukleinsäure C hybridisieren kann. Die Bedingungen der Hybridisierungen der Zielnukleinsäure mit der Sondennukleinsäure und der Matrizennukleinsäure mit dem Spaltprodukt B' können unabhängig gewählt werden.

Die erfindungsgemäße Matrizennukleinsäure C enthält einen Teil C2, der mit dem Spaltprodukt B' und insbesondere dessen nukleinsäurespezifischen Teil B2 oder einem Teil davon und gegebenenfalls eventuell noch vorhandener Reste von B1 hybridisieren kann. Als Matrizennukleinsäure C sind alle Moleküle geeignet, die eine solche Hybridisierung erlauben, d.h. Nukleinsäuren, die aus den natürlichen Nukleotidbausteinen aufgebaut sind, jedoch auch Nukleinsäureanaloge, die aus nicht natürlich vorkommenden Bausteinen aufgebaut sind oder solche Bausteine enthalten. Darüberhinaus sollte die Matrizennukleinsäure stabil gegenüber Abbau unter den gewählten Bedingungen sein. Für den Fall, daß die Spaltung der Sondennukleinsäure mit RNAse vorgenommen wird, handelt es sich bei der Matrizennukleinsäure besonders bevorzugt um Deoxyribonukleinsäure. Neben dem Teil C2 weist die Matrizennukleinsäure einen Teil C1 auf, der nicht mit dem Teil B1 bzw. noch vorhandenen Resten des Teils B1 der Sondennukleinsäure allein hybridisieren kann. Besonders bevorzugt bildet dieser Teil C1 auch mit nicht nachzuweisenden Nukleinsäuren in der Probe und der Zielnukleinsäure selbst keine unter den gewählten Bedingungen stabilen Hybride aus. Bevorzugt liegt der Teil C1 in 5'-Richtung, gesehen vom Teil C2.

Die Matrizennukleinsäure bzw. der nukleinsäurespezifische Teil B2 werden bevorzugt so gewählt, daß das 5'-Ende des Spaltprodukts B' bzw. der Sondennukleinsäure im hybridisierten Zustand nicht über das 3'-Ende der Matrizennukleinsäure hinausragt, wenn in einer nachgeschalteten Reaktion der Einbau von Desoxynukleotiden nachgewiesen wird. Dies soll eine enzymatische Verlängerung der Matrizennukleinsäure verhindern. Denselben Effekt kann man erreichen, indem die Matrizennukleinsäure an ihrem 3'-Ende gegen enzymatische Verlängerung geschützt ist, z.B. durch Einsatz eines nicht verlängerbaren Mononukleotids.

In jedem Fall sollte gewährleistet sein, daß das oder die Spaltprodukte B' mit der Matrizennukleinsäure derartig hybridisieren können, daß B' auch an dem durch Spaltung entstandenen Ende mit der Matrizennukleinsäure hybridisiert und ein Teil C1 der Matrizennukleinsäure im hybridisierten Zustand noch über das durch Spaltung entstandene Ende des Spaltprodukts B' hinausragt.

In einem anschließenden Schritt wird das Hybrid aus Spaltprodukt B' und Matrizennukleinsäure C bestimmt, wobei die Bestimmung so durchgeführt wird, daß eventuell gebildete Hybride aus ungespaltener Sondennukleinsäure B und Matrizennukleinsäure C nicht bestimmt werden.

Eine dieser Möglichkeiten beruht darauf, daß das Spaltprodukt B' unter Verwendung der Matrizennukleinsäure C verlängert wird, während die ungespaltene Sondennukleinsäure B nicht verlängert wird. Dies beruht auf der oben beschriebenen strukturellen Eigenschaft der Matrizennukleinsäure, nicht mit dem Teil B1 der Sondennukleinsäure zu hybridisieren, während das Spaltprodukt B' mit der Matrizennukleinsäure hybridisiert. Spaltprodukt B' hybridisiert insbesondere an der Spaltstelle, (am 3'-Ende von B') mit C. Da das Ende der Matrizennukleinsäure über das Ende des Spaltprodukts hinaussteht, kann in einer Verlängerungsreaktion ein Nukleinsäureteil, welcher zu dem entsprechenden Teil der Matrizennukleinsäure C komplementär ist, angehängt werden. Für eine solche Verlängerungsreaktion existieren eine Reihe von Möglichkeiten. Eine dieser Möglichkeiten ist das Anhängen von Mononukleotiden mit Hilfe einer DNA-Polymerase. In einem bevorzugten Fall sind die eingesetzten Mononukleosidtriphosphate markiert, z.B. gemäß EP-A-0 324 474. Wenn die Mononukleosidtriphosphate nicht markiert sind, kann die Bildung des Verlängerungsprodukts beispielsweise über eine Auftrennung mit Gelelektrophorese nachgewiesen werden. In einer besonderen Ausführungsform können auch zwei unterschiedliche Markierungen eingebaut werden. Dann kann über eine Markierung eine Immobilisierung der gebildeten Verlängerungsprodukte und über die andere Markierung ein Nachweis durchgeführt werden. Hierfür sind beispielsweise die in EP-A-0 437 774 beschriebenen Bedingungen möglich.

In einer weiteren Ausführungsform dient Spaltprodukt B' als Primer in einer Polymerasekettenreaktion. Die Sondennukleinsäure B kann in ungespaltenem Zustand nicht als Primer fungieren. Hierbei ist das Templat der PCR die Matrizennukleinsäure C. Bei Verwendung zweier verschiedener Sondennukleinsäuren B, das heißt wenn diese an verschiedene Bereiche zueinander komplementärer Zielnukleinsäure hybridisieren, können beide Primer einer PCR erzeugt werden.

Eine weitere Möglichkeit, das Spaltprodukt B' zu verlängern, ist mittels der Ligase Reaction möglich. Hierzu wird ein Oligonukleotid zugesetzt, welches mit der Matrizennukleinsäure so hybridisiert, daß zwischen dem hybridisierten Spaltprodukt B'und diesem Oligonukleotid ein Nick verbleibt, welcher mit Hilfe einer Ligase geschlossen werden kann.

In einer weiteren bevorzugten Ausführungsform enthält der Teil C1 der Matrizennukleinsäure eine Transkriptions- oder Replikations-initiierende Sequenz. Bevorzugt enthält C1 eine Promotersequenz. Durch die matrizenkontrollierte Verlängerung des Spaltprodukts B' wird ein funktioneller, vollständiger Promotor gebildet. Nach Initiierung einer Transkription, die nur stattfinden kann, wenn die Verlängerung tatsächlich stattgefunden hat, kann die Entstehung von Spaltprodukten und somit der Anwesenheit der Zielnukleinsäure nachgewiesen werden. Hierzu wird die Reaktionsmischung mit allen für die Transkription erforderlichen Reagenzien versetzt, wozu eine auf den Promotor abgestimmte RNA Polymerase, z.B. T7 RNA Polymerase sowie Ribonukleosidtriphosphate, von denen ein Teil bevorzugt eine Markierung trägt, gehören. Die Anwesenheit der Transkripte kann dann zum Nachweis der Zielnukleinsäure verwendet werden. Reagenzien und Bedingungen, wie sie für eine in-vitro-Transkription erforderlich sind, sind beispielsweise der EP-A-0 329 822 zu entnehmen. Bei der nachgeschalteten Transkription wird im Vergleich mit der ersten Ausführungsform eine höhere Sensitivität erreicht.

Die Temperatur des erfindungsgemäßen Verfahrens wird so gewählt, daß die Aktivitäten der eingesetzten Enzyme möglichst optimal sind und gleichzeitig die Hybridisierungsbedingungen ausreichende Spezifität erlauben. Bei der Verwendung nicht thermostabiler RNAse ist beispielsweise ein Temperaturbereich zwischen 30° und 60°, besonders bevorzugt 42° zweckmäßig. Bei Einsatz einer thermostabilen RNAse sind höhere Temperaturen möglich. Für die Verlängerungsreaktion richtet sich die Temperatur ebenfalls nach dem für die Verlängerung eingesetzten Enzym. Dabei ist bei der Verwendung von thermostabilen Enzymen, z.B. Taq-DNA-Polymerase ein Bereich zwischen 50° und 80° bevorzugt und besonders bevorzugt ca. 60-70°. Bei Verwendung eines transkribierenden Enzyms richtet sich die optimale Temperatur ebenfalls nach der Aktivität des Enzyms, z.B. für T3, T7 und SP6-Polymerase zwischen 25° und 45°, besonders bevorzugt 37°. Durch Erhöhung der Aufenthaltswahrscheinlichkeit der Enzyme an den Reaktionsorten dürfte es möglich sein, die Umsatzraten zu steigern und somit die erforderliche Reaktionszeit zu senken oder die Sensitivität zu erhöhen.

Die Matrizennukleinsäure kann auch ein circuläres Molekül darstellen. Für diesen Fall ist die Verwendung eines Verlängerungsenzyms mit Strangverdrängungsaktivität bevorzugt.

Die hohe Flexibilität und mögliche Doppelmarkierung ermöglichen die Anwendung des Verfahrens auf Teststreifen oder den Nachweis über Kopplung an Beads und deren Nachweis in Durchflußcytometrie oder Kapillarelektrophorese.

In einer möglichen Ausführungsform enthält ein Teil der Matrizennukleinsäure eine Sequenz, die als Matrize für die Synthese einer replizierbaren RNA dient, z.B. MDV 1 RNA oder kürzere Sequenzen.

Die Sondennukleinsäure und die Matrizennukleinsäure können nach prinzipiell bekannten Verfahren hergestellt werden, sobald die Sequenz ihrer Teile festgelegt ist. Für den bevorzugten Fall, daß es sich um Oligonukleotide mit einer Länge von weniger als 100 Mononukleotidbausteinen handelt, ist die Synthese nach geläufigen chemischen Verfahren (z.B. Festphasensynthese analog Merrifield) bevorzugt. Dies ermöglicht auch die einfache Synthese gemischter Oligonukleotide (RNA/DNA-Chimäre). Für größere Nukleinsäuren sind rekombinante Herstellverfahren oder chemisch/enzymatische Verfahren, wie in EP-A-325 970 beschrieben, bevorzugt. Teil B1 ist bevorzugt 12-35, Teil B2 bevorzugt 15-50, Teil C1 bevorzugt 10-100 und Teil C2 15-50 Nukleotide lang.

Figur 1 zeigt eine erste Ausführungsform der Erfindung, die auf dem Einbau nicht radioaktiv markierter Deoxyribonukleotidtriphosphate durch eine DNA Polymerase beruht.

Figur 2 zeigt schematisch eine Ausführungsform, bei der das Matrizenoligonukleotid eine Promotorsequenz enthält, die zu einem funktionellen Promotor gemacht wird und anschließende Transkription. Die Promotorsequenz ist schraffiert dargestellt.

In Figur 3 ist die Sensitivität des Verfahrens nach Figur 1 in Abhängigkeit von der Menge der Sondennukleinsäure und bei einer Menge von 10 pmol Matrizennukleinsäure pro Ansatz dargestellt.

In Figur 4 bis 6 ist die Spezifität eines Nachweises von DNA-Sequenzen im Verfahren gemäß Figur 1 in Abhängigkeit von der Inkubationstemperatur der Hybridisierungs- und Abbaureaktion gezeigt (42°C, 50°C und 56°C).

In Figur 7 ist die Sensitivität des Nachweises gemäß dem Verfahren von Figur 2 dargestellt.

In Figur 8 ist schematisch eine Ausführungsform gezeigt, bei der Sondennukleinsäure und Matrizennukleinsäure miteinander verbunden sind.

Die in den Figuren gezeigten Schritte können nacheinander unter Zugabe der jeweils benötigten Reagenzien durchgeführt werden. Alle benötigten Komponenten können bei entsprechendem Design der Komponenten jedoch auch schon zu Beginn der Reaktion zugesetzt werden, so daß der Prozeß simultan abläuft.

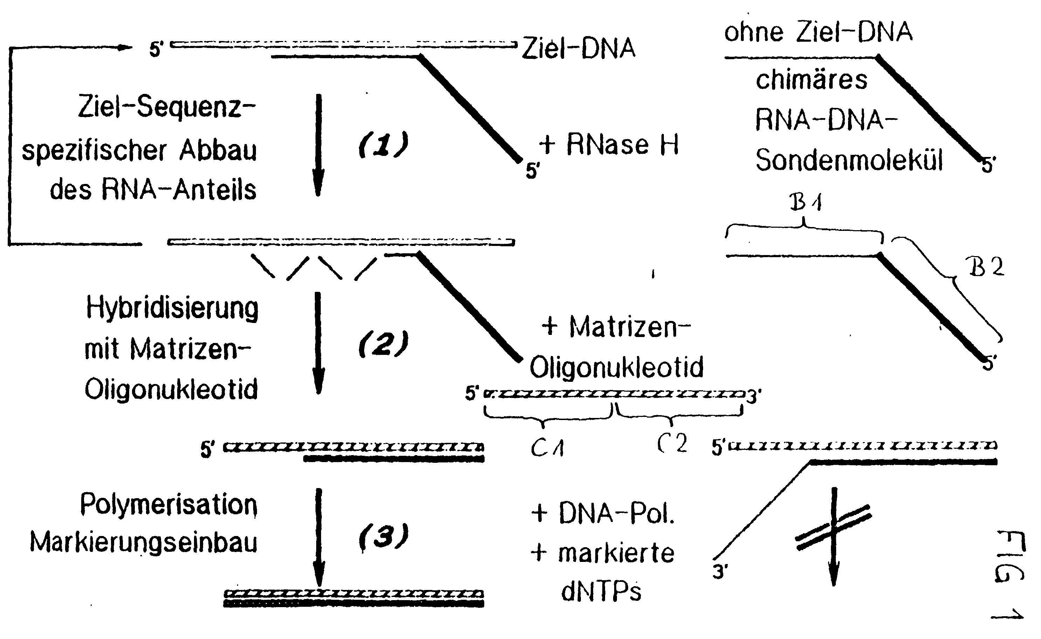

In einer ersten bevorzugten Ausführungsform (FIG 1) wird eine Probe, die eine Ziel-DNA enthält, mit einem chimären RNA-DNA- oder DNA-RNA-DNA-Sondenmolekül versetzt, so daß eine Hybridisierung stattfindet. Durch RNAse H wird der Zielsequenz-spezifische RNA-Anteil der Sonde abgebaut. Danach wird das Matrizenoligonukleotid unter Einstellen geeigneter Hybridisierungsbedingungen zusammen mit einer DNA-Polymerase und unmarkierten und markierten Deoxyribonukleosidtriphosphaten zugegeben. Der Einbau markierter Mononukleotide kann beispielsweise nach Abtrennung der Nukleinsäuren von den nicht umgesetzten markierten Deoxyribonukleosidtriphosphaten nachgewiesen werden. Die Verwendung zweier unterschiedlich markierter Nukleotide ermöglicht den direkten festphasengebundenen Nachweis. Wenn in der Probe keine Ziel-DNA enthalten ist, hybridisiert das Matrizenoligonukleotid nur mit dem chimären RNA-DNA-Sondenmolekül. Eine Verlängerung des Sondenmoleküls kann nicht stattfinden, da das 3'-Ende nicht mit dem Matrizenoligonukleotid hybridisiert.

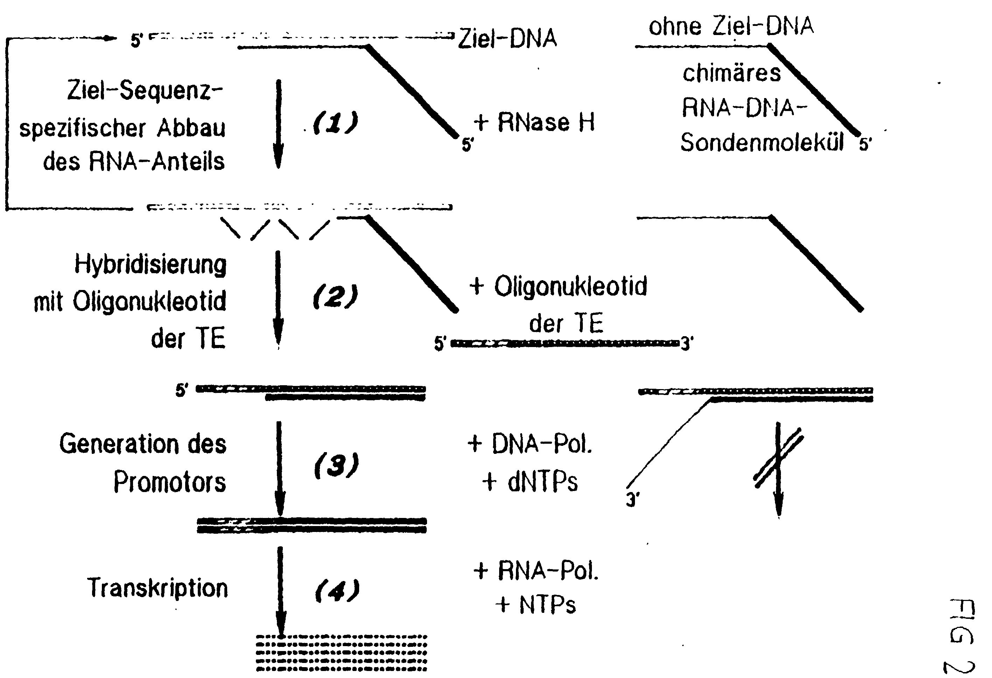

In einer zweiten, bevorzugten Ausführungsform (Figur 2) wird ebenfalls zunächst die Probe mit der Ziel-DNA mit dem chimären RNA-DNA-Sondenmolekül oder DNA-RNA-DNA-Sondenmolekül zur Hybridisierung gebracht. Nach Abbau des Zielsequenz-spezifischen RNA-Anteils durch RNAse H wird das Spaltprodukt mit einem Oligonukleotid zur Hybridisierung gebracht, welches an seinem 5'-Ende über das Ende des Spaltprodukts hinausragt und eine einzelsträngige Promotorsequenz enthält. Durch Zugabe einer DNA Polymerase und Deoxyribonukleotidtriphosphaten wird das 3'-Ende des Spaltproduktes so verlängert, daß sich ein funktioneller Promotor bildet. Die entstandene doppelsträngige Nukleinsäure wird einer Transkription durch RNA Polymerase und Nukleosidtriphosphate unterzogen. Promoter-Sequenzen sind dem Fachmann für verschiedene Polymerasen bekannt (z.B. Nucl. Acids. Res. 12, 7035-7056 (1984)).

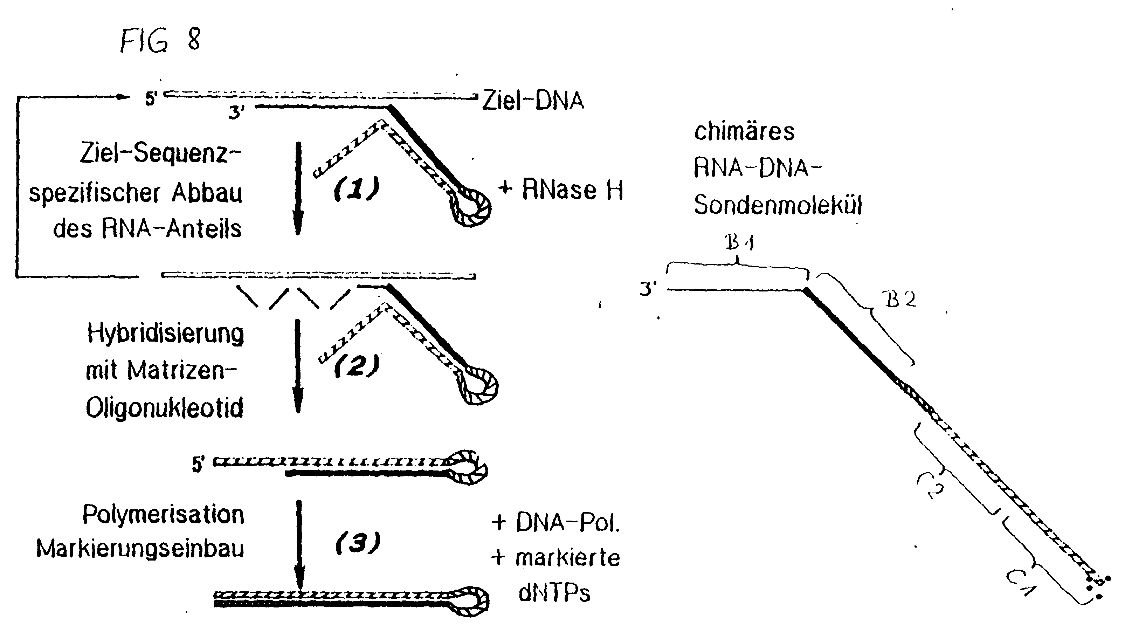

In einer dritten Ausführungsform (Figur 8) sind die Sondennukleinsäure und die Matrizennukleinsäure keine getrennten Moleküle, sondern miteinander verknüpft. Hierzu wird ein Ende der Matrizennukleinsäure an das von B1 wegzeigende Ende des Teils B2 der Sondennukleinsäure so angehängt, daß Teil C2 mit Teil B2 unter Bildung einer Haarnadelstruktur hybridisieren kann, und die Teile B1 und C1 die Enden des Haarnadelmoleküls darstellen. Die Verknüpfung der Teile B2 und C2 kann beliebig geschehen, beispielsweise durch kovalente Verknüpfung, bevorzugt durch Mononukleotideinheiten, z. B. Oligo-dA. Damit sich die Haarnadelstruktur bilden kann ist es empfehlenswert, zwischen den miteinander hybridisierbaren Teilen C2 und B2 einen Linker, der 3 bis 20 Nukleotide oder Äquivalente lang ist, vorzusehen. Für den Fall, daß B2 am 5'-Ende des Sondenmoleküls lokalisiert ist, findet die Verknüpfung zwischen dem 5'-Ende von B2 und dem 3'-Ende von C2 statt. Diese Ausführungsform hat den Vorteil, daß durch die räumliche Nähe der miteinander hybridisierenden Teile der Sondennukleinsäure und der Matrizennukleinsäure eine Erhöhung der Reaktionsgeschwindigkeit erreicht werden kann.

Die in Figur 8 geschilderte Ausführungsform ist analog auch auf Figur 2 zu übertragen (Generation eines Promotors).

Ein Vorteil des erfindungsgemäßen Verfahrens ist es, daß die Sondennukleinsäure B keine Markierung enthalten muß. Dies erleichtert die Synthese der Sondennukleinsäure, führt jedoch auch dazu, daß ein Hintergrundsignal über diese Markierungen in einer späteren Nachweisreaktion nicht auftreten kann. Bei dem erfindungsgemäßen Verfahren wird nicht eine Zielsequenz amplifiziert. Daher kann die Gefahr der Kontamination mit amplifizierten Molekülen aus vorangegangenen Versuchen ausgeschlossen werden. Bei dem erfindungsgemäßen Verfahren dient das Produkt des letzten Teilschrittes nicht als Substrat für die erste Teilreaktion. Aus diesem Grund ist der Ablauf der Reaktion leichter kontrollierbar. Dadurch, daß die Amplifikation im wesentlichen linear ist, ist eine quantitative Auswertung erleichtert. Zum anderen ist die Gefahr falsch positiver Ergebnisse durch Kreuzkontamination ursprünglich negativer Proben mit geringen Mengen wirklich positiver Proben gegenüber den exponentiellen Amplifikationssystemen vermindert. Das erfindungsgemäße Verfahren benötigt keine Gerätschaften für eine zyklische Variation der Inkubationstemperatur. Das erfindungsgemäße Verfahren ist auch insofern flexibel, als durch Einsatz zweier unterschiedlicher nicht radioaktiver Markierungen gemäß EP-A-0 437 774 sowohl eine Wandbindung als auch ein Nachweis der Produkte möglich ist. Da im erfindungsgemäßen Verfahren der weitere Ablauf der Teilreaktionen von der spezifischen Hybridisierung des Sondenmoleküls mit der Zielsequenz unbedingt abhängig ist, können keine Hintergrundsignale durch unspezifische Bindung oder durch Persistenz nicht hybridisierter Sondenmoleküle entstehen, wie sie beispielsweise bei Amplifikation mit Hilfe replizierbarer Ribonukleinsäuresequenzen (Qβ-Replikation) auftreten. Im Gegensatz zu Verfahren, bei denen zwei oder mehr Oligonukleotide unterschiedlicher Sequenz in einem Ansatz zur Reaktion gebracht werden, kann eine unspezifische Reaktion der Oligonukleotide miteinander (wie z.B. Primer-Dimere in der PCR) beim erfindungsgemäßen Verfahren nahezu ausgeschlossen werden. Das erfindungsgemäße Verfahren kann bei nicht stringenten Hybridisierungsbedingungen durchgeführt werden. Dadurch wird der Nachweis von verwandten Sequenzen möglich. Die Durchführung unter sehr stringenten Bedingungen ermöglicht den Nachweis von Punktmutationen. Dadurch, daß der Nachweis im erfindungsgemäßen Verfahren nicht über den Nachweis eines markierten verkürzten Sondenmoleküls neben den ursprünglich eingesetzen markierten Sondenmolekülen ablaufen muß, wird die Verwendung gelelektrophoretischer Methoden zur Trennung dieser Sequenzen vermieden. Dadurch wird das Verfahren automatisierbar und somit für die Routineanalytik einsetzbar.

Ebenfalls Gegenstand der Erfindung ist ein Reagenzkit zur Durchführung des oben genannten Nachweisverfahrens. Dieser Kit enthält die Sondennukleinsäure B und getrennt oder im gleichen Behälter die Matrizennukleinsäure C. Bevorzugt enthält das Reagenzkit außerdem Puffer für die Durchführung der Hybridisierungs- und Abbaureaktion bzw. der Verlängerungsreaktion. Besonders bevorzugt enthält der Kit nachweisbar markierte Mononukleosidtriphosphate. Soweit die Reagenzien auf zwei verschiedene Behälter aufgeteilt sind, enthält der erste Behälter bevorzugt die Sondennukleinsäure, ein Enzym für die Durchführung der Abbaureaktion sowie geeignete Puffer. Im zweiten Behälter befinden sich dann die Matrizennukleinsäure, sowie mindestens ein Enzym für die Durchführung der Verlängerungsreaktion mit Mononukleosidtriphosphaten, sowie einen geeigneten Puffer.

Die folgenden Beispiele sollen die Erfindung näher erläutern.

### Beispiel 1

### Verfahren unter Einbau markierter Mononukleotide

Im Folgenden werden in Oligonukleotiden Großbuchstaben für Deoxyribonukleotideinheiten und Kleinbuchstaben für Ribonukleotideinheiten verwendet.

### Vorgehensweise:

1 pmol, 10 pmol oder 100 pmol des RNA-DNA-Sondenmoleküls (LPR03_19R:
5'-GATCGGACTGGAAGTAATACGACTCACcgccgcgucgcagaagauc-3' (SEQ. ID. NO. 1)
oder
LPR03_25R:
5'-GATCGGACTGGAAGTAATACGACTCACcgccgcgucgcagaagaucucaauc-3' (SEQ. ID NO. 2) wurden mit jeweils unterschiedlichen Mengen der nachzuweisenden DNA (DHBV1: 5'-GATTGAGATCTTCTGCGACGCGGCGGT-3' (SEQ. ID. NO. 3)) in einem Volumen von 10 µl bei 42°C für 3 h in Puffer P2 (10 mM Hepes, 1mM MgCl₂, pH 8,0) unter Zusatz von 3 µg RSA, 20 U RNasin und 4 U RNase H inkubiert. Dann wurden 10 pmol des Matrizenoligonukleotides
TAQ (5'-ATTCCATGATTCATTGATTATTGTCGCGGCGGTGAGTCGT ATTACTTCCAGTCCGATC-3' (SEQ. ID. NO 4)) zugegeben, der Ansatz für 1 min auf 100°C erhitzt und sofort auf Eis abgekühlt. Für die Auffüllreaktion wurden die Ansätze mit 2 µl Taq-Puffer (10 mM Tris pH 8,3, 50 mM KCl,l 1,5 mM MgCl₂, 0,1 mg/ml Gelatine), der Nukleotid-Mischung 1_3 (je 1 µM dATP, dGTP, dCTP, DIG-dUTP (Boehringer Mannheim) und BIO-dUTP (Boehringer Mannheim)), 20 U RNasin, 1 U Taq-DNA-Polymerase und H₂O auf 20 µl aufgefüllt und bei 60°C für 15 min inkubiert.

Für den Nachweis wurden die Reaktionsansätze mit Puffer D (10 mM HEPES (pH 8,0), 30 mM NaCl, 1 mM MnCl₂ auf je 210 µl verdünnt. Die BIO-DIG-markierten Syntheseprodukte wurden in einer Streptavidin-beschichteten Mikrotiterplatte (MTP) immobilisiert (TRSA-SA-MTP, Firma MicroCoat), indem je zwei mal 100 µl des mit Puffer D verdünnten Reaktionsansatzes in die Vertiefungen einer mit Waschpuffer (0,5 % (V/V) Tween 20 in PBS ("phosphate buffered saline")) vorgewaschenen SA-MTP pipettiert wurden. Die MTP wurde unter Schütteln (Well-Warml, Fa. Denley Instruments GmbH) für 1 h bei 37°C inkubiert.

Die MTP mit den immobilisierten Nukleinsäuremolekülen wurde fünf mal mit je 200 µl Waschpuffer gewaschen. Dann wurden je 100 µl der Konjugatverdünnung (polyklonale <DIG>-S-F_{ab}-POD_{poly}-Konjugate (Boehringer Mannheim GmbH), 200 mU/ml in Konjugatpuffer (100 mM Na-Phosphat (pH 7,5), 0,9 % (G/V) NaCl, 1 % (G/V) Ralufon F4J oder RSA Fraktion V; der Konjugatpuffer wurde mit DEPC behandelt, sterilfiltriert (0,2 µm-Filter, Nalgene) und bei 4°C aufbewahrt)) zugegeben und erneut unter denselben Bedingungen inkubiert.

Ungebundene Konjugatmoleküle wurden durch fünfmaliges Waschen entfernt. Pro Vertiefung wurden nun 100 µl der Substratlösung (2,2'-azino-di-[3-ethylbenzthiazolinsulfat(6)], ABTS) zugegeben. Die Farbreaktion erfolgte bei 37°C unter Schütteln. Die "Optische Dichte" des umgesetzten ABTS bei 405 nm wurde nach kurzem Schütteln der MTP unmittelbar vor der Messung mittels eines ELISA-Readers (SLT) gegen den Referenzfilter von 492 nm gemessen und nach Abzug des Nullwertes (nur ABTS) die Mittelwerte der Doppelbestimmungen ermittelt (SLT Easy-Base Version 4.01).

### Sensitivität

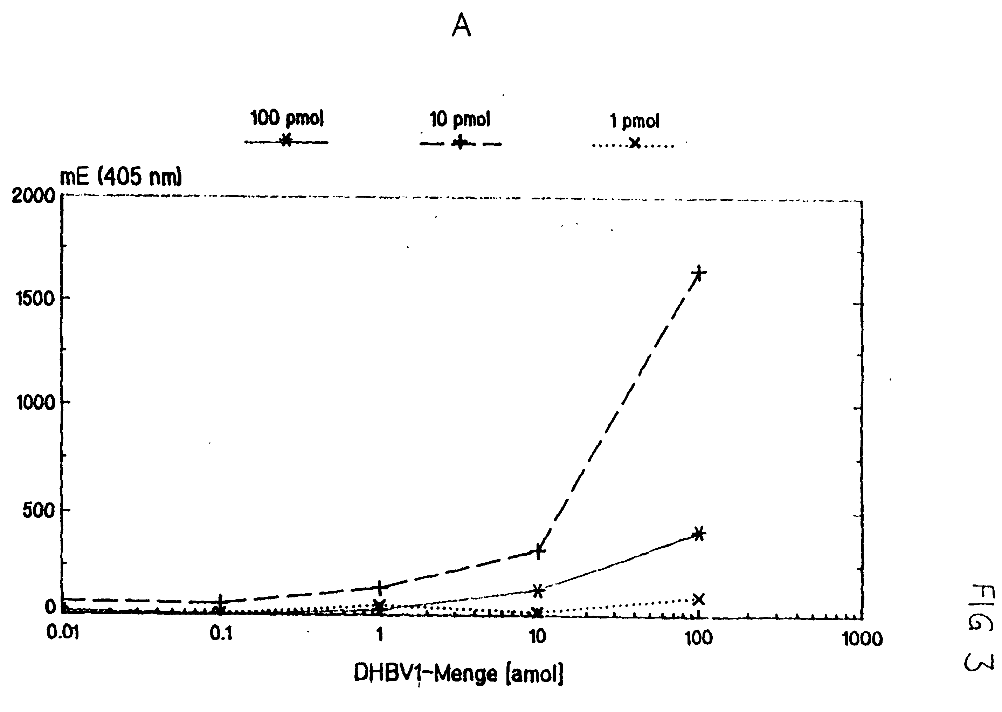

Zur Ermittlung der Sensitivität des Verfahrens wurde im oben geschilderten Reaktionsansatz die Matrizennukleinsäure in einer Konzentration von 10 pmol eingesetzt. Das Ergebnis ist in Figur 3 gezeigt. Die Kurven in Abbildung 3 führten zu dem Ergebnis, daß unter den gewählten Reaktionsbedingungen (10 pmol Matrizenoligonukleotid, Menge an RNase H, Inkubationsvolumen, Inkubationstemperatur usw.) der sensitivste Nachweis mit 10 pmol des Sondenmoleküls erzielt wurde. Der Einsatz von nur 1 pmol Sondennukleinsäure begrenzt wahrscheinlich den ersten Teilschritt der Reaktion, die Hybridisierung- und Abbaureaktion. Werden 100 pmol Sondennukleinsäure eingesetzt, können nicht alle abgebauten Sondenmoleküle mit einem Matrizenoligonukleotid hybridisieren und dadurch verlängert werden. Es läßt sich also festhalten, daß unter den gewählten Bedingungen die größte Sensitivität mit 10 pmol Sondennukleinsäure pro Ansatz erreicht wurde und daß unter diesen Bedingungen 10 amol Zielnukleinsäure nachgewiesen werden konnten. Diese Nachweisgrenze liegt um etwa den Faktor 10 niedriger als in der Methode gemäß WO 89/10415, in der unter äquivalenten Bedingungen 0,1 fmol der Zielnukleinsäure detektiert wurden.

### Temperaturabhängigkeit

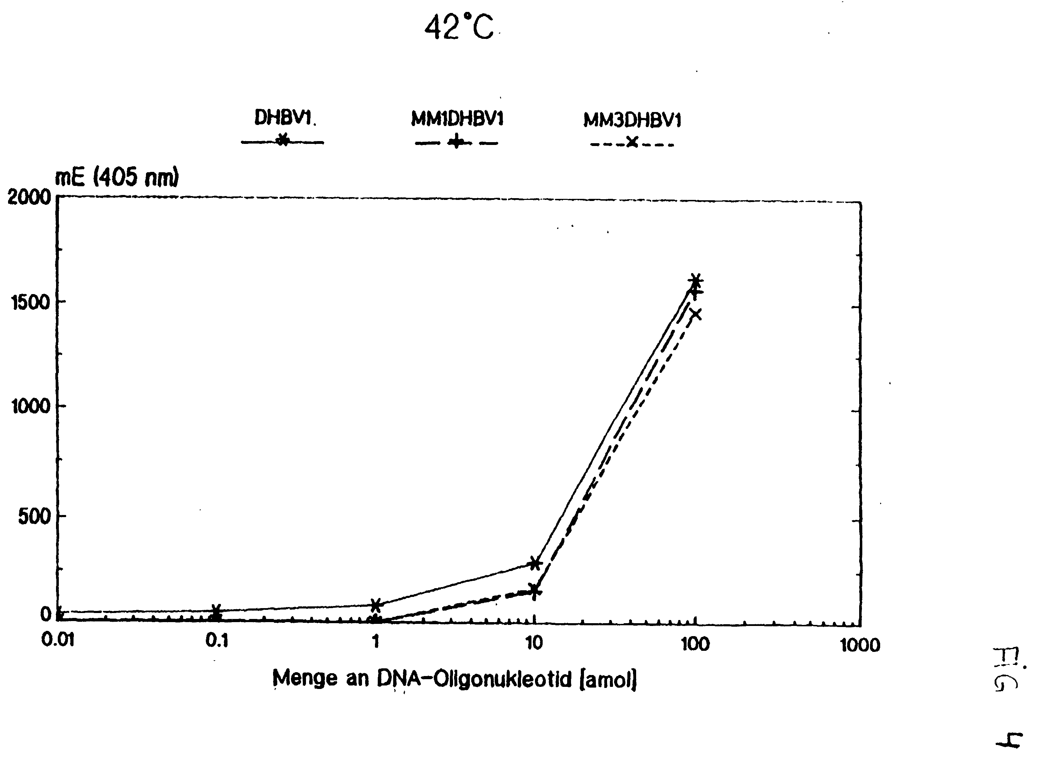

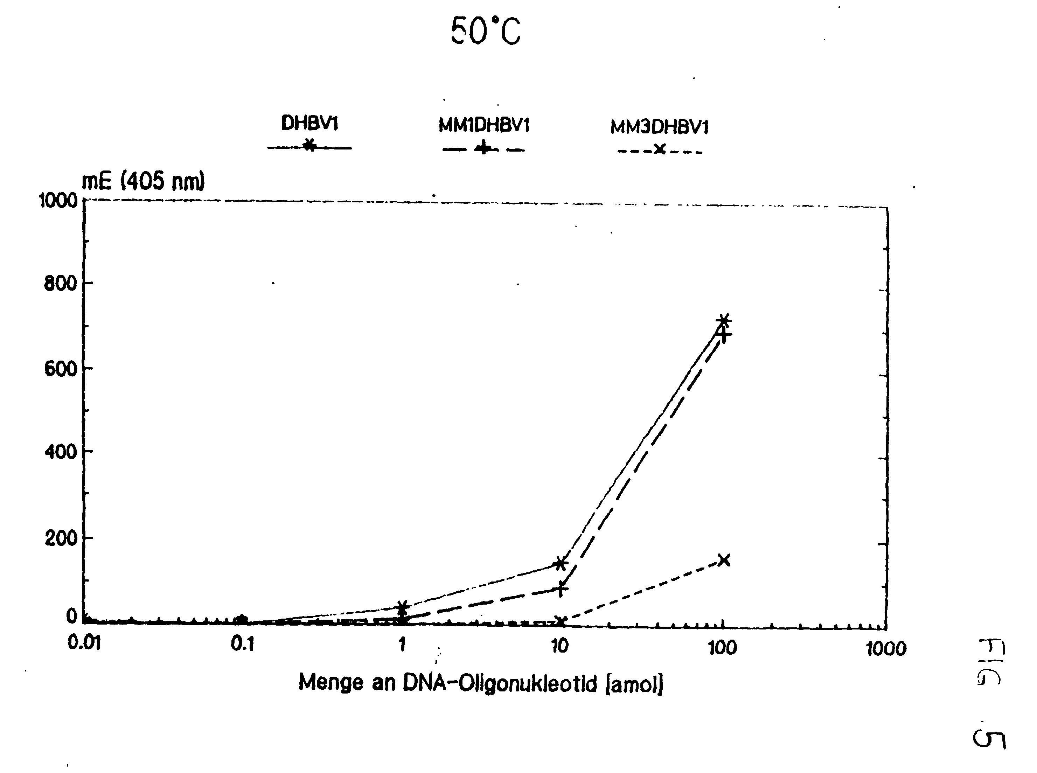

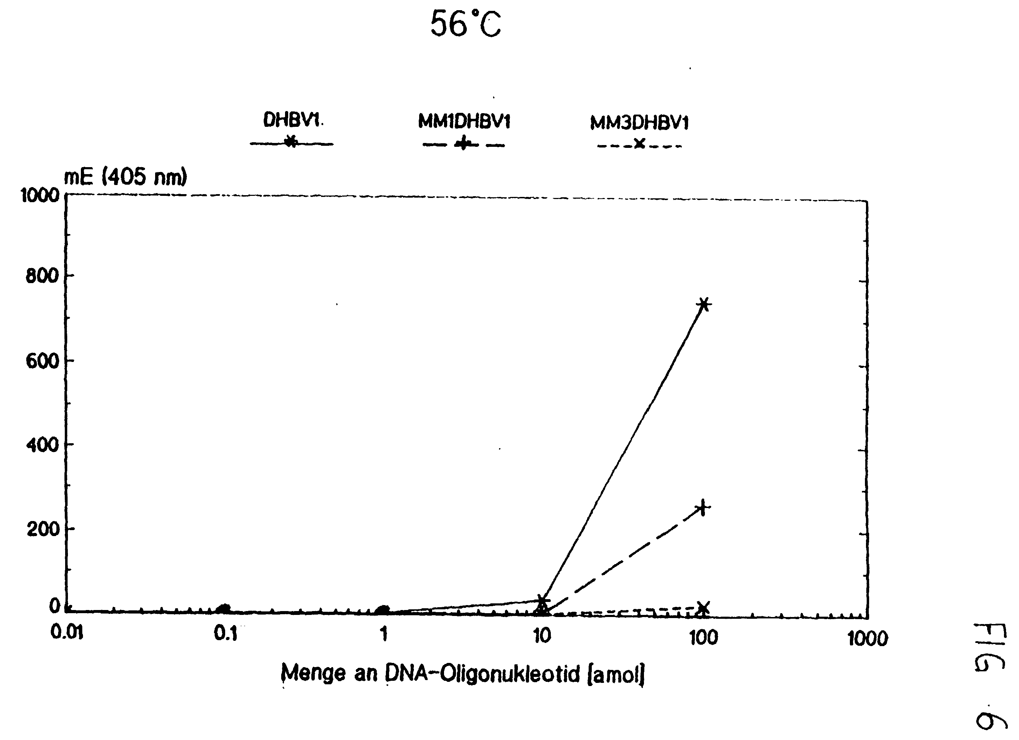

Zur Beschreibung der Temperaturabhängigkeit des erfindungsgemäßen Verfahrens von Beispiel 1 wurden pro Ansatz 10 pmol Sondennukleinsäure eingesetzt und in Gegenwart von je 0,01 amol bis 100 amol Zielnukleinsäure mit RNAse H bei 42°C (Figur 4), 50°C (Figur 5) bzw. 56°C (Figur 6) inkubiert. Die sich anschließende Auffüllreaktion fand in allen Fällen bei 60°C statt. Die Messung erfolgte nach 30minütiger Inkubation mit ABTS. Als Sondennukleinsäure wurde HBV 1 bzw.
MM 1 DHBV 1 (5'-GATTGAGATCTTATGCGACGCGGCGGT-3', SEQ. ID. No. 7) (eine Basenfehlpaarung) bzw. MM 3 DHBV 1 (5'-GATTGAGATCTCACG CGACGCGGCGGT-3', SEQ. ID. No. 8) (drei Basenfehlpaarungen) eingesetzt. Bei einer Inkubationstemperatur der Abbaureaktion von 42°C führte der Versuch zu fast identischen Kurvenverläufen und somit zu einer geringen Sequenzspezifität des erfindungsgemäßen Verfahrens. Die Erhöhung der Stringenz durch die Anhebung der Inkubationstemperatur auf 50°C ergab für den Nachweis des perfekt komplementären Oligonukleotids die HBV 1 und des eine Basenfehlpaarung ausbildenden Oligonukleotids ähnliche Kurvenverläufe, allerdings mit einem Sensitivitätsverlust gegenüber der Inkubationstemperatur von 42°C. Der Grund hierfür lag in dem weniger vollständigen Abbau des RNA-Anteils. Deutlich geringere Signale erzeugte das drei Basenfehlpaarungen ausbildenden Oligonukleotid, was auf die geringere Hybridisierungsrate unter den stringenteren Bedingungen zurückzuführen war. Die größte Spezifität wurde bei einer Inkubationstemperatur von 56°C erreicht. Hier riefen 100 amol DHBV 1 ein deutliches Signal hervor, 100 amol MM 1 DHBV 1 führten zu einem niedrigeren Wert der optischen Dichte und 100 amol MM 3 DHBV 1 ließen sich nicht nachweisen. Damit ist gezeigt, daß mit dem erfindungsgemäßen Verfahren ein sequenzspezifischer Nachweis von Nukleinsäuren möglich ist. In 3x3 Serien wurden 10 pmol Sondennukleinsäure mit unterschiedlichen Mengen (0,01 amol bis 100 amol) der perfekt komplementären Zielnukleinsäure DHBV 1, der eine zentrale Basenfehlpaarung ausbildenden Zielnukleinsäure MM 1 HBV 1 und der drei zentrale Basenfehlpaarungen ausbildenden Zielnukleinsäure MM 3 DHBV 1 eingesetzt. Die ersten drei Serien wurden bei 42°C, die zweiten bei 50°C und die dritten bei 56°C inkubiert. Als Negativkontrollen dienten jeweils Ansätze ohne Zielnukleinsäure. Die Auffüllreaktion fand in allen Fällen bei 60°C statt. Die in der sich anschließenden Nachweisreaktion in einer mit Streptavidin gecoateten Mikrotiterplatte gemessenen Werte sind in Figur 4 bis 6 graphisch dargestellt.

### Beispiel 2

### Verfahren unter Bildung eines funktionellen Promotors

### Vorgehensweise:

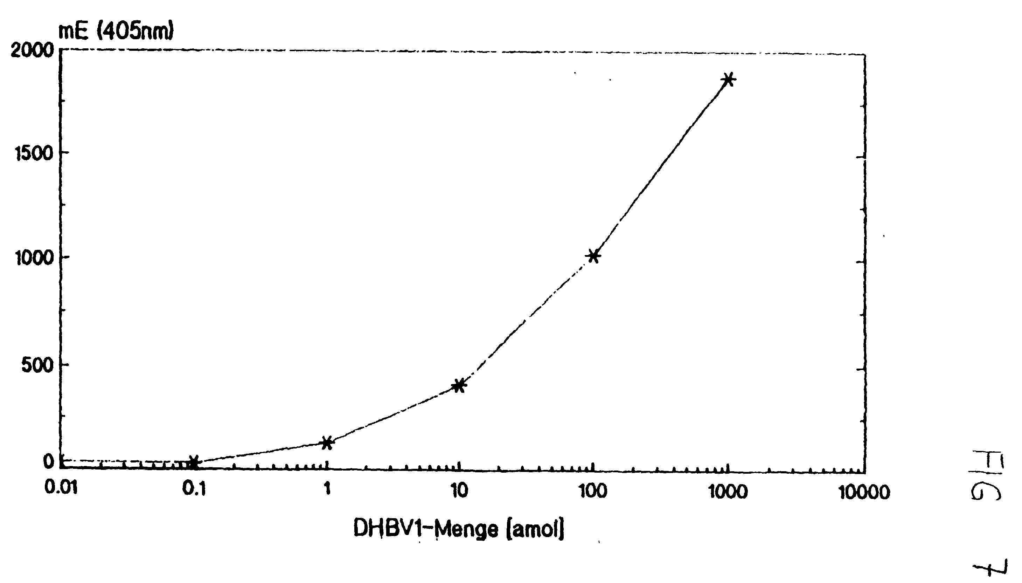

Je 10 pmol RDCPTAQ
(5'-ATGATAGTTGATGATAGTTGGATATcgccgccuggcagaagauc-3' (SEQ. ID. NO. 5))
wurden mit unterschiedlichen Mengen DHBV1 (5'-GATTGAGATCTTCT
GCGACGCGGCGGT-3' (SEQ. ID. NO. 5)), 0,01 amol bis 10 finol) in einem Volumen von 10 µl mit RNase H für 1 h bei 42°C Puffer P2 (10 mM Hepes, 1 mM MgCl₂, pH 8,0) unter Zusatz von 3 µg RSA, 20 U RNasin und 4 U RNase H inkubiert. Als Negativkontrollen diente ein Ansatz ohne DHBV1. Dann wurde jeder Ansatz mit 10 pmol QAT2 (5'-GATCGGACTGGAAGTAATACGACTCACTATAGGGCGGCGA TATCCAACTATCATCAACTATCAT-3' (SEQ. ID. NO. 6)), 2 µl Taq-Puffer (10 mM Tris pH 8,3, 50 mM KCl, 1,5 mM MgCl₂, 01, mg/ml Gelatine) und je 1 mM dATG, dGTP, dCTP und dTTP versetzt und die Taq-DNA-Polymerase-katalysierte Auffüllreaktion für 15 min bei 60°C unter Erhöhung des Reaktionsvolumens auf 20 µl durchgeführt. Anschließend wurden je 3 µl Transkriptionspuffer (40 mM Tris pH 7,9, 6 mM MgCl₂, 10 mM DTT, 2 mM Spermidin), 20 U RNasin, 0,5 µl 1 % Triton X-100 und 500 U T7-RNA-Polymerase zugesetzt und als Nukleotidmischung die Mischung CP1 mit DIG- und BIO-UTP eingesetzt (9,84 µM ATP, 4,92 µM GTP, 2,46 µM CTP, 3,28 µM UTP, 820 nM DIG-UTP und 820 nM BIO-UTP); das Gesamtvolumen betrug nun 30 µl. Die Inkubation erfolgte bei 37°C für 2 h. Für die Detektionsreaktion wurden alle Ansätze mit Puffer D auf 210 µl verdünnt und je zweimal 100 µl in die Vertiefungen einer SA-MTP pipettiert. Der Nachweis erfolgte mittels <DIG>-PODpoly und ABTS (Boehringer Mannheim) wie in Beispiel 1 beschrieben; die gemessenen Werte sind in Figur 7 graphisch dargestellt.

Wie aus dem Kurvenverlauf hervorgeht, konnten unter den gewählten Reaktionsbedingungen 1 amol der Zielsequenz DHBV 1 nachgewiesen werden. Diese Nachweisgrenze liegt um etwa den Faktor 10 niedriger als in dem Verfahren unter Einbau markierter Desoxyribonukleotide (Beispiel 1), in dem unter äquivalenten Bedingungen 10 amol der Zielnukleinsäure detektiert wurden.

## Patentansprüche

1. Verfahren zum empfindlichen Nachweis einer Ziel-Nukleinsäure A durch
- Hybridisierung der Zielnukleinsäure A mit einer Sondennukleinsäure B, die einen mit der Zielnukleinsäure hybridisierbaren Teil B 1 und einen möglichst nicht mit der Zielnukleinsäure hybridisierenden nukleinsäurespezifischen Teil B2 enthält,
- Spaltung der Sondennukleinsäure B im Teil B1,
- Hybridisierung eines Spaltproduktes B' der Sondennukleinsäure, welches den nicht mit der Zielnukleinsäure hybridisierenden Teil B2 enthält, mit einer Matrizennukleinsäure C, die einen mit dem Spaltprodukt im Teil B2 hybridisierbaren Teil C2 und einen nicht mit dem Teil B1 der Sondennukleinsäure hybridisierbaren Teil C1 enthält und
- Bestimmung des Hybrids aus Spaltprodukt B' und Matrizennukleinsäure C.

2. Verfahren gemäß Anspruch 1, **dadurch gekennzeichnet, daß** das Spaltprodukt B' unter Verwendung der Matrizennukleinsäure C verlängert wird, während die Sondennukleinsäure nicht verlängert wird.

3. Verfahren gemäß Anspruch 2, **dadurch gekennzeichnet, daß** die Matrizennukleinsäure C in Teil C1 eine Promotorsequenz enthält.

4. Verfahren gemäß Anspruch 2, **dadurch gekennzeichnet, daß** die Verlängerung unter Verwendung nicht-radioaktiver markierter Mononukleotide geschieht.

5. Verfahren gemäß Anspruch 1, **dadurch gekennzeichnet, daß** der Teil C1 in 5'-Richtung vom Teil C2 gelegen ist.

6. Verfahren gemäß Anspruch 2, **dadurch gekennzeichnet, daß** die Matrizennukleinsäure an ihrem 3'-Ende gegen Verlängerung geschützt ist.

7. Verfahren gemäß Anspruch 2, **dadurch gekennzeichnet, daß** das 5'-Ende des Spaltprodukts B' bzw. der Sondennukleinsäure im hybridisierten Zustand nicht über das 3'-Ende der Matrizennukleinsäure hinausragt.

8. Verfahren gemäß Anspruch 1, **dadurch gekennzeichnet, daß** die Zielnukleinsäure nach Spaltung der Sondennukleinsäure erneut mit einem Sondennukleinsäuremolekül hybridisiert wird.

9. Reagenzkit zum Nachweis einer Zielnukleinsäure enthaltend
- eine Sondennukleinsäure B, die einen mit der Zielnukleinsäure hybridisierbaren Teil B1 und einen nicht mit der Zielnukleinsäure hybridisierenden nukleinsäurespezifischen Teil B2 enthält,
- eine Matrizennukleinsäure C, die einen mit der Sondennukleinsäure in Teil B2 hybridisierenden Teil C2 und
- einen nicht mit Teil B1 der Sondennukleinsäure hybridisierbaren Teil C1 enthält,
- ein Enzym zur matrizenabhängigen Synthese von Nukleinsäuren und
- geeignete Puffer.

## Claims

1. Method for the sensitive detection of a target nucleic acid A by
- hybridizing the target nucleic acid A with a probe nucleic acid B which contains a part B1 that can hybridize with the target nucleic acid and a nucleic acid-specific part B2 that preferably does not hybridize with the target nucleic acid,
- cleaving the probe nucleic acid B in part B1,
- hybridizing a cleavage product B' of the probe nucleic acid which contains part B2 that does not hybridize with the target nucleic acid with a template nucleic acid C which contains a part C2 that can hybridize with the cleavage product in part B2 and a part C1 which does not hybridize with part B1 of the probe nucleic acid, and
- determining the hybrid of cleavage product B' and template nucleic acid C.

2. Method as claimed in claim 1, **characterized in that** the cleavage product B' is extended using the template nucleic acid C while the probe nucleic acid is not extended.

3. Method as claimed in claim 2, **characterized in that** the template nucleic acid C contains a promoter sequence in part C1.

4. Method as claimed in claim 2, **characterized in that** it is extended using non-radioactively labelled mononucleotides.

5. Method as claimed in claim 1, **characterized in that** part C1 is located in the 5' direction from part C2.

6. Method as claimed in claim 2, **characterized in that** the template nucleic acid is protected against extension at its 3' end.

7. Method as claimed in claim 2, **characterized in that** the 5' end of the cleavage product B' or of the probe nucleic acid does not protrude beyond the 3' end of the template nucleic acid in the hybridized state.

8. Method as claimed in claim 1, **characterized in that** after the probe nucleic acid has been cleaved, the target nucleic acid is again hybridized with a probe nucleic acid molecule.

9. Reagent kit for the detection of a target nucleic acid comprising
- a probe nucleic acid B which contains a part B1 that can hybridize with the target nucleic acid and a nucleic acid-specific part B2 that cannot hybridize with the target nucleic acid,
- a template nucleic acid C which contains a part C2 that can hybridize with the probe nucleic acid in part B2 and
- contains a part C1 that cannot hybridize with part B1 of the probe nucleic acid,
- an enzyme for the template-dependent synthesis of nucleic acids, and
- a suitable buffer.

## Revendications

1. Procédé pour la caractérisation sensible d'un acide nucléique cible A par
- hybridation de l'acide nucléique cible A avec un acide nucléique B utilisé comme sonde qui contient une partie B1 apte à s'hybrider avec l'acide nucléique cible et une partie 82 manifestant une spécificité pour un acide nucléique qui, pour autant que possible, ne s'hybride pas avec l'acide nucléique cible,
- coupure de l'acide nucléique B utilisé comme sonde dans la partie B1,
- hybridation d'un produit de coupure B' de l'acide nucléique utilisé comme sonde, qui contient la partie B2 qui ne s'hybride pas à l'acide nucléique cible, avec un acide nucléique C faisant office de matrice qui contient une partie C2 apte à s'hybrider avec le produit de coupure dans la partie B2 et une partie C1 inapte à s'hybrider avec la partie B1 de l'acide nucléique utilisé comme sonde, et
- détermination de l'hybride constitué par le produit de coupure B' et par l'acide nucléique C faisant office de matrice.

2. Procédé selon la revendication 1, **caractérisé en ce qu'**on prolonge le produit de coupure B' en utilisant l'acide nucléique C faisant office de matrice, tandis que l'on ne prolonge pas l'acide nucléique utilisé comme sonde.

3. Procédé selon la revendication 2, **caractérisé en ce que** l'acide nucléique C faisant office de matrice contient une séquence de promoteur dans la partie C1.

4. Procédé selon la revendication 2, **caractérisé en ce que** le prolongement a lieu en utilisant des mononucléotides non radiomarqués.

5. Procédé selon la revendication 1, **caractérisé en ce que** la partie C1 est disposée dans la direction 5' par rapport à la partie C2.

6. Procédé selon la revendication 2, **caractérisé en ce que** l'acide nucléique faisant office de matrice est protégé contre le prolongement à son extrémité 3'.

7. Procédé selon la revendication 2, **caractérisé en ce que** l'extrémité 5' du produit de coupure B', respectivement de l'acide nucléique utilisé comme sonde, à l'état hybridé ne fait pas saillie au-delà de l'extrémité 3' de l'acide nucléique faisant office de matrice.

8. Procédé selon la revendication 1, **caractérisé en ce que** l'acide nucléique cible est une nouvelle fois hybridé, après la coupure de l'acide nucléique utilisé comme sonde, avec une molécule d'acide nucléique utilisée comme sonde.

9. Nécessaire pour la caractérisation d'un acide nucléique cible contenant
- un acide nucléique B utilisé comme sonde qui contient une partie B1 apte à s'hybrider avec l'acide nucléique cible et une partie B2 manifestant une spécificité pour un acide nucléique qui, pour autant que possible, ne s'hybride pas avec l'acide nucléique cible,
- un acide nucléique C faisant office de matrice qui contient une partie C2 qui s'hybride avec l'acide nucléique utilisé comme sonde dans la partie B2 et
- une partie C1 inapte à s'hybrider avec la partie B1 de l'acide nucléique utilisé comme sonde,
- une enzyme pour la synthèse d'acides nucléiques en fonction de la matrice et
- des tampons appropriés.


### SEQUENZPROTOKOLL

(1) ALLGEMEINE INFORMATION:
   (i) ANMELDER:
      (A) NAME: Boehringer Mannheim GmbH
      (B) STRASSE: Sandhoferstr. 116
      (C) ORT: Mannheim
      (E) LAND: DE
      (F) POSTLEITZAHL: 68305
      (G) TELEPHON: 06217594348
      (H) TELEFAX: 06217594457
   (ii) ANMELDETITEL: Verfahren zum sensitiven Nachweis von Nukleinsaeuren
   (iii) ANZAHL DER SEQUENZEN: 8
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 46 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: misc_feature
      (B) LAGE: 1..46
      (D) SONSTIGE ANGABEN: /note= "Nukleotide 28-46 sind
         Ribonukleotide, Nukleotide 1-27 sind
         Deoxyribonukleotide"
   (xi) SEQUENZBESCHREIBUNG: SEQ ID NO: 1:
   GATCGGACTG GAAGTAATAC GACTCACCGC CGCGUCGCAG AAGAUC 46
(2) INFORMATION ZU SEQ ID NO: 2:
   (i) SEQUENZ CHARAKTERISTIKA:
      (A) LÄNGE: 52 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: misc_feature
      (B) LAGE: 1..52
      (D) SONSTIGE ANGABEN: /note= "Nukleotide 28-52 sind
         Ribonukleotide, Nukleotide 1-27 sind
         Deoxyribonukleotide"
   (xi) SEQUENZBESCHREIBUNG: SEQ ID NO: 2:
(2) INFORMATION ZU SEQ ID NO: 3:
   (i) SEQUENZ CHARAKTERISTIKA:
      (A) LÄNGE: 27 Basenpaare
      (B) ART: Nukleinsäure
      (C) STRANGFORM: Einzel
      (D) TOPOLOGIE: linear
   (ii) ART DES MOLEKÜS: DNS (genomisch)
   (xi) SEQUENZBESCHREIBUNG: SEQ ID NO: 3:
   GATTGAGATC TTCTGCGACG CGGCGGT 27
(2) INFORMATION ZU SEQ ID NO: 4:
   (i) SEQUENZ CHARAKTERISTIKA:
      (A) LÄGE: 58 Basenpaare
      (B) ART: Nukleinsäure
      (C) STRANGFORM: Einzel
      (D) TOPOLOGIE: linear
   (ii) ART DES MOLEKÜS: DNS (genomisch)
   (xi) SEQUENZBESCHREIBUNG: SEQ ID NO: 4:
(2) INFORMATION ZU SEQ ID NO: 5:
   (i) SEQUENZ CHARAKTERISTIKA:
      (A) LÄNGE: 45 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: misc_feature
      (B) LAGE: 1..45
      (D) SONSTIGE ANGABEN: /note= "Nukleotide 27-45 sind
         Ribonukleotide, Nukleotide 1-26 sind
         Deoxyribonukleotide"
   (xi) SEQUENZBESCHREIBUNG: SEQ ID NO: 5:
   ATGATAGTTG ATGATAGTTG GATATACGCC GCCUGGCAGA AGAUC 45
2) INFORMATION ZU SEQ ID NO; 6:
   (i) SEQUENZ CHARAKTERISTIKA:
      (A) LÄNGE: 64 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: promoter
      (B) LAGE: 15..31
   (xi) SEQUENZBESCHREIBUNG: SEQ ID NO: 6:
(2) INFORMATION ZU SEQ ID NO: 7:
   (i) SEQUENZ CHARAKTERISTIKA:
      (A) LÄNGE: 27 Basenpaare
      (B) ART: Nukleinäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: mutation
      (B) LAGE: replace(13,"")
      (D) SONSTIGE ANGABEN: /note= "Unterschied zu SEQ.ID.NO. 3"
   (xi) SEQUENZBESCHREIBUNG: SEQ ID NO: 7:
   GATTGAGATC TTATGCGACG CGGCGGT 27
(2) INFORMATION ZU SEQ ID NO: 8:
   (i) SEQUENZ CHARAKTERISTIKA:
      (A) LÄNGE: 27 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: mutation
      (B) LAGE: replace(12..14,"")
      (D) SONSTIGE ANGABEN: /note= "Unterschiede zu SEQ.ID.NO.3"
   (xi) SEQUENZBESCHREIBUNG: SEQ ID NO: 8:
   GATTGAGATC TCACGCGACG CGGCGGT 27